# EUROPEAN PATENT SPECIFICATION

(11) **EP 1 117 460 B1**
(45) Date of publication and mention of the grant of the patent: **12.01.2005**
(21) Application number: 99950119.0
(22) Date of filing: 01.10.1999
(51) Int. Cl.: A61M 37/00

(54) **COMPONENT MIXING CATHETER**
KATHETER, DER DAS MISCHEN VON KOMPONENTEN ERMÖGLICHT
CATHETER DE MELANGE DE COMPOSANTS

(30) Priority: 01.10.1998 US 102636 P
(43) Date of publication of application: 25.07.2001
(73) Proprietor: BAXTER INTERNATIONAL INC., Deerfield, IL 60015 (US)
(72) Inventor: EPSTEIN, Gordon, Howard, Fremont, CA 94539 (US); LEVINSON, Mitchell, E., Pleasanton, CA 94588 (US)
(74) Representative: Crowhurst, Charlotte Waveney
(86) International application number: PCT/US1999/022964
(87) International publication number: WO 2000/018469

(56) References cited:
- EP-A- 0 424 068
- US-A- 4 170 232
- US-A- 4 735 619
- US-A- 5 030 201
- US-A- 5 217 441
- US-A- 5 322 510
- US-A- 5 443 454
- US-A- 5 626 562
- US-A- 5 779 721
- US-A- 5 814 022
- US-A- 5 975 367
- US-A- 5 989 215

## Description

### BACKGROUND OF THE INVENTION

### 1. Field of the Invention

The present invention relates to an apparatus for applying component parts of a sealant which when mixed transforms from a fluidic state to a non-fluidic state. In particular but not exclusively, the present invention is directed to an apparatus in which sealant components are mixed prior to being applied to biological tissue to effect hemostasis or achieve other therapeutic results.

### 2. Description of Related Art

Use of tissue sealants and other biological materials is an important emerging surgical technique, well adapted for the operating room or field environments such as the doctor's office or mobile medical units. In addition, the application of such sealants via a catheter provides a non evasive medical technique. Preferred sealants include fibrin sealants which are formed from blood plasma components and comprise, on the one hand, a first component containing fibrinogen and Factor XIII and on the other hand a second component which usually includes thrombin, and calcium ions.

The fibrinogen is capable of a polymerizing and being cross-linked to form a solid tibrin clot when the components are mixed. The necessary additional factors to simulate relevant portions of the natural blood coagulation cascade are suitably distributed between the fibrinogen and thrombin components.

High levels of protection against transmission of infections or induction of immunological reactions can be assured by using an autologous or single-donor source for both components. Such sealants are highly effective, are biologically degraded without residue and may promote wound healing.

Depending upon the potency of the particular formulations employed, coagulation of the sealant may take place very rapidly, yielding a get within perhaps 10 or 20 seconds after mixing of the two components. Though often very desirable for surgical reasons, such fast-acting properties present potential problems of fouling or clogging. These problems must be overcome in devising suitable applicators, and methods of application.

A popular manually operable applicator for such two-component sealants employs a dual syringe construction wherein two syringes, connected by yoke, each provide a reservoir for one of the components. In most prior devices, the sealant components are discharged in separate streams and mixed externally of the applicator. Such applicators are similar in principle to household epoxy glue applicators commonly available in hardware stores. Achieving effective mixing externally of the applicator is problematic.

In United States patent No. 5, 266,877, and the above applications, the present inventor teaches various constructions of a dual syringe applicator wherein the fluid sealant components are mixed internally.

Antanavich et al. United States Patent number 5,585,007 provides an extensive discussion of the literature relating to fibrinogen sealant preparation (column 1, line 20 to column 4. line 62) and applicators column 4 line 62 to column 5, line 14), as well as a bibliography, (columns 6.10) and is a helpful guide to the teachings of prior workers in the field.

In one or more of the above copending applications the possibility of retrograde clearing of the mixed fluids pathway within the applicator, using suction, is also disclosed. The applicator is provided with suitable suction conduits and valving to apply suction to the work surface, to prepare it for the application of sealant, for example by removing fluids. As taught, the valving is operable to effect retrograde clearing of a sealant dispensing pathway. Enhanced mixing is possible for example, by impingement and problems of fouling by deposited solids are avoided.

Such applicators, and methods, are remarkably effective for applying sealants to exposed biological surfaces. However, it would be desirable to provide a surgeon, or other user, with additional choices, for example, an applicator and method that could effectively apply sealant to internal biological locations.

One difficulty is that the coagulating nature of the sealants causes the discharge opening or openings of an application device to become clogged so that flow out of the applicator slows down or stops.

US-A-5,443,454 describes an embolectomy catheter containing a lumen for the introduction of a liquid substance and provided in the distal and thereof with spinning means for extruding the liquid substance in the form of a filamentous embolic material.

While the above-referenced copending applications disclose effective clearing-enabled sealant application devices and methods, their techniques are generally intended for application of sealant to exposed and accessible work surfaces.

There is accordingly a need for a sealant applicator and method that can be used to reach an unexposed or inaccessible location.

### SUMMARY OF THE INVENTION

The present invention solves this problem by providing a sealant applicator comprising a dual catheter communicating with fluid sealant agent sources, for example, two internal reservoirs, which sealant applicator can effectively deliver multiple sealant components to a remote tip of the catheter for mixing and dispensing to an area of application. This catheter is a dual catheter having one catheter movably mounted with respect to another, for example longitudinally within the other. The movable catheter is then used as a plunger to unclog the openings.

The present invention enables an effective sealant composition to reach an area of application by mixing the sealant components to application or contact with the work surface and providing for removal of coagulated sealant.

Preferably, although not necessarily, the sealant is a biological sealant, for example a tissue adhesive, and the area of application is a biological tissue subject to surgery. The sealant components can comprise a first, structural component capable of gelling, and preferably of solidification and a second, activation component which activates such gelling and, optionally, solidification. More preferably, the sealant is a tissue sealant and the first component comprises fibrinogen and the second component comprises, or can generate a fibrinogen activator, especially thrombin or an equivalent thereof.

### BRIEF DESCRIPTION OF THE DRAWINGS

One way of carrying out the invention is described in detail below with reference to the drawings which illustrate one or more specific embodiments of the invention and in which:
- **Figure 1**: is a cross sectional elevational view of a catheter sealant applicator according to the present invention;
- **Figure 2**: is a complete bottom view along lines 2-2 of Figure 1;
- **Figure 3a**: is a view similar to Figure 1 illustrating operation of the applicator of the present invention;
- Figure 3b: is a cross sectional view along the lines 3b-3b of Figure 3a:
- Figure 3c: is a cross sectional view of an alternative embodiment of the present invention;
- **Figure 4**: is a cross sectional elevational of an alternative embodiment of the present invention;
- **Figure 5**: is a cross sectional view illustrating operation of the present invention;
- **Figure 6**: is a cross sectional view illustrating operation of the present invention;
- **Figure 7**: is a cross sectional view illustrating operation of the present invention;
- **Figure 8**: is a cross sectional view illustrating operation of the present invention;
- **Figure 9**: is a perspective view of a component part of the present invention;
- **Figures 10 and 10a**: are a cross sectional view illustrating an alternative embodiment of the present invention;
- **Figures 11 and 11a**: are a cross sectional view illustrating an alternative embodiment of the present invention;
- **Figure 12**: is a cross sectional view illustrating an alternative embodiment of the present invention;
- **Figure 13**: is a cross sectional view illustrating an alternative embodiment of the present invention;
- **Figure 14**: is a cross sectional view illustrating an alternative embodiment of the present invention;
- **Figure 15**: is a cross sectional view illustrating an alternative embodiment of the present invention;
- **Figure 16**: is a perspective view of a further embodiment of the invention employing a plug-like inner catheter, in a retracted position within an outer catheter shown as being transparent to reveal the inner structure; and
- **Figure 17**: is a view similar to Figure 16 with the plug-like inner catheter in an advanced position.

### DESCRIPTION OF THE PREFERRED EMBODIMENTS

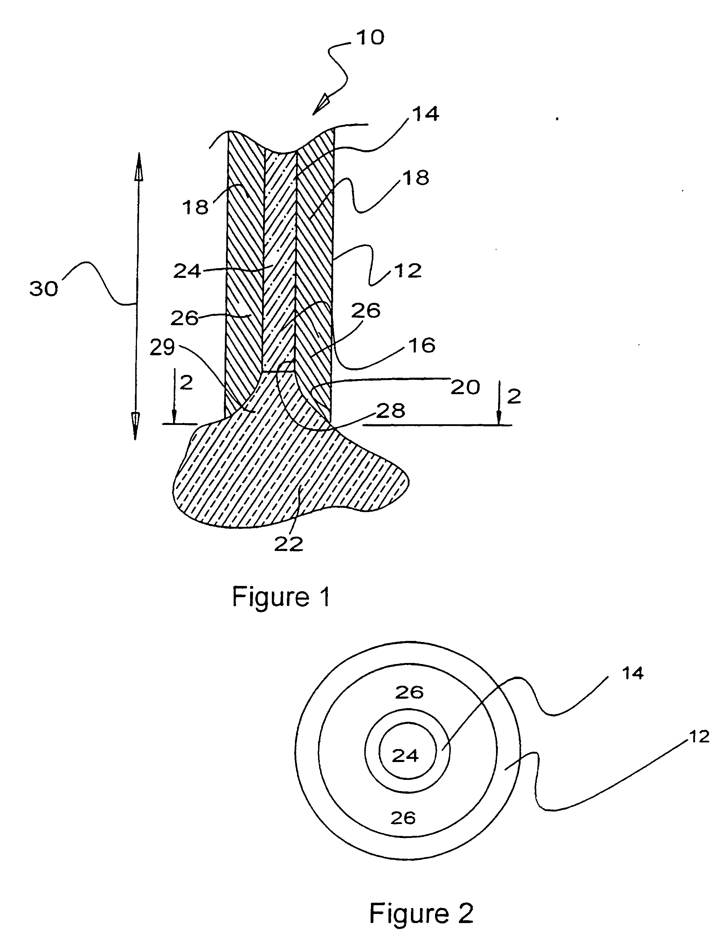

Referring now to Figures 1 and 2, a sealant applicator 10 is illustrated. Applicator tO comprises a pair of inner and outer coaxial catheters 12 and 14. In the preferred embodiment inner and outer catheters 12 and 14 are elongated tubes. Inner and outer catheters 12 and 14 are constructed out of any suitable, preferably sterilizable material, for example, stainless steel or polypropylene, and may be rigid or flexible, or may comprise both rigid and flexible components along their lengths. Typically, catheters are substantially longer than the dispensing cannulas described in preferred embodiments of the parent application, having, for example, a length of at least 10 cm, often at least 20 cm and sometimes being substantially longer, for example a meter or more. Commonly, catheters are flexible, or have a flexible portion.

Inner catheter 14 defines an area 16 and is of a smaller diameter than outer catheter 12, this allows outer catheter 12 to surround inner catheter 14 leaving a second area 18.

Outer catheter 12 is configured to have an opening 20 for dispersing a mixed sealant 22.

Sealant 22 is typically comprised of a first sealant agent or component 24 and a second agent or component 26. Component 24 is contained within area 16 and component 26 is contained with area 18. Inner catheter 14 has an opening 28 for discharge of component 24, and in the position shown in Figure 1 is retracted behind opening 20 of outer catheter 12 to provide a mixing volume 29.

In the preferred embodiment areas 16 and 18 and their respective ratios are of such a configuration as to provide desired proportions of components 24 and 26 to provide a sealant 22 in accordance with the required parameters. As may be desired, areas 16 and 18 and their corresponding volumes may be varied to provide a sealant 22 of various characteristics. For instance, a sealant that may require a longer time to cure or a sealant that may require lesser time to cure by using a lesser or greater proportion or concentration of activator, e.g. thrombin for a fibrinogen sealant.

Areas 16 and 18 are of sufficient size to allow for unimpeded flow of the sealant components.

Inner catheter 14 is slidably or otherwise movably mounted with respect to outer catheter 12 to permit relative movement of inner catheter 14 in the directions of arrow 30.

The movement of outer catheter 12 allows opening 28 of inner catheter 14 to be positioned within outer catheter 12 and away from opening 20. This position allows components 24 and 26 to mix in an area in close proximity to opening 20. Moreover, and as illustrated in Figure 3a, the position of opening 28 may be further retracted with respect to outer catheter 12. This positioning will provide a larger mixing volume 29 allowing for a longer mixing time of component 24 and 26.

Alternatively, opening 28 may be positioned closer to opening 20, if desired, to reduce the volume of mixing volume 29 and the time components 24 and 26 are in contact with each other inside applicator 10. This may be appropriate for a very fast setting sealant.

To stabilize inner catheter 14 and maintain the appropriate mixing volumes of components 24 and 26, a stabilizing ring 27 (as illustrated by the dashed lines in Figure 3a) or the equivalent thereof can be affixed to inner catheter 14 and extend outwardly to outer catheter 12. Stabilizing ring 27 provides stability to inner catheter 14 and is configured to allow component 26 to flow through the same.

Alternatively, and depending on the length of catheters 12 and 14, a plurality of stabilizing rings 27 may positioned within applicator 10 to provide stability to inner catheter 14.

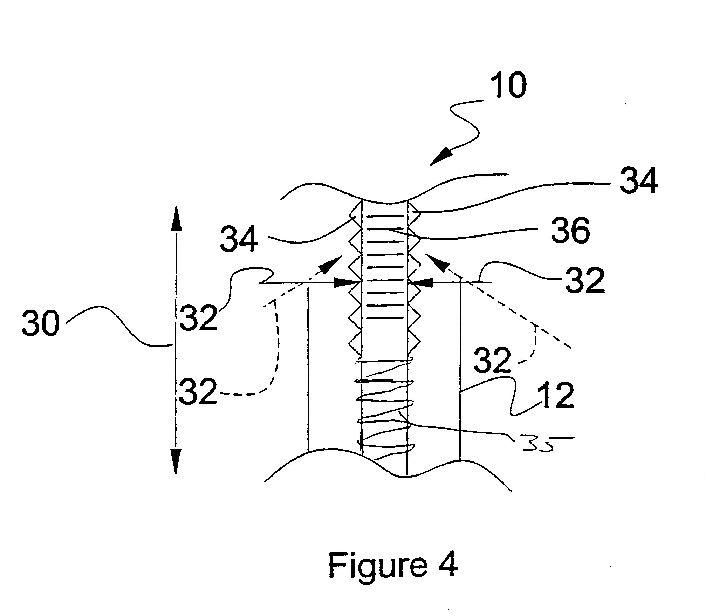

The movement of inner catheter 14 may be effected by any suitable mechanism for example, a proximally located ratchet and pawl mechanism as illustrated in Figure 4. In this embodiment a locking mechanism 32 restricts the movement of the proximal end of inner catheter 14 by engaging with a plurality of nubs 34. Movement of mechanism 32 into a position, as illustrated by the dashed lines in Figure 4 will once more allow inner catheter 14 to move in the direction of arrow 30. Mechanism 32 may be configured to have a conveniently placed trigger to facilitate the release.

In addition, and as an alternative, mechanism 32 can work in conjunction with a return spring 35 which is depressed as inner catheter 14 is depressed. Accordingly, as mechanism 32 is released the force of spring 35 acts to return inner catheter 14 to its original position.

Optionally, inner catheter 14 may be provided with graduated markings 36. Markings 36 can indicate the relative position of opening 28 of inner catheter 14 with respect to opening 20 of outer catheter 12. For instance, a marking of zero would indicate that opening 28 and opening 20 are flush with each other. Other markings such as +1, +2, +3, or - 1, -2, -3 would indicate the respective location of opening 28 with respect to 20.

In yet another embodiment markings 36 can indicate the flow output of sealant 22 depending on the position of opening 28. Depending on the size of catheters 12 and 14, markings 36 can be configured to indicate flow output of mixed sealant 22, such as millimeters per second or any other suitable indicator of flow output. In addition, markings 36 may also be configured to refer to the mixture ratio of component 24 with respect to component 26.

The movement of inner catheter 14 with respect to outer catheter 12 also allows a user to conveniently purge any clot of hardened sealant 22 from opening 20, as will now be further explained.

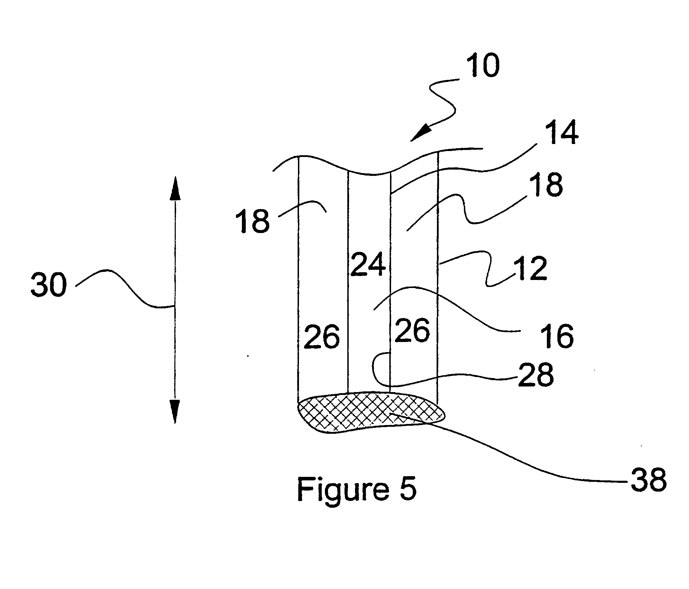

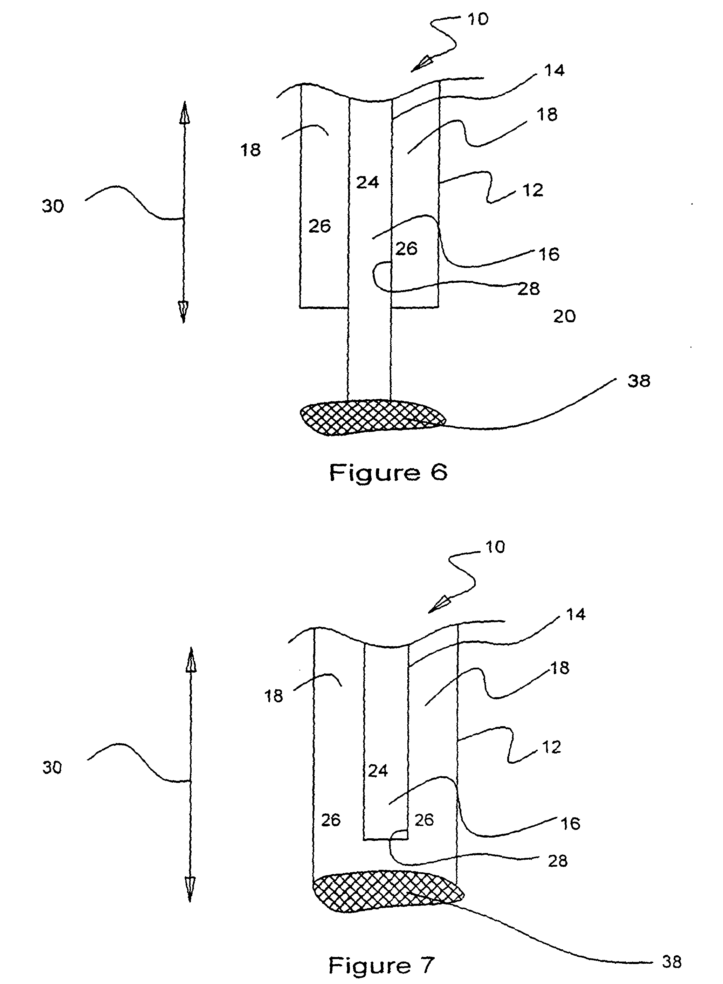

Turning now to Figures 5 and 6, and as previously discussed the internal mixing of components 24 and 26 effects coagulation of sealant 22. However, such coagulation may cause a clot 38 to block opening 20. This problem is addressed by the mobility of inner catheter 14. To facilitate the removal of clot 38 the user can simply move inner catheter 14 to the position illustrated in Figure 6. Such movement may be accomplished through the use of a trigger mechanism as described with reference to Figure 4, or any other suitable mechanism.

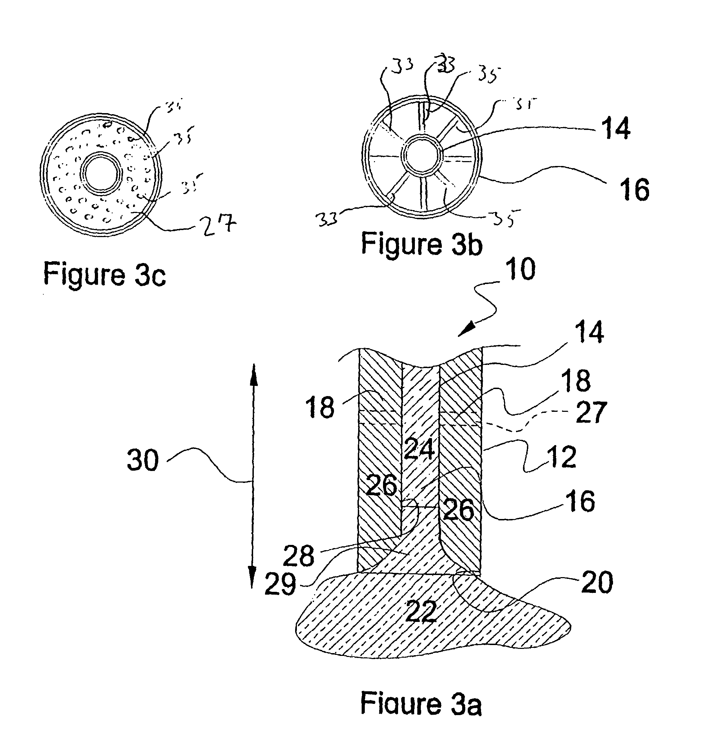

In yet another embodiment stabilizing ring 27 (Figures 3a and 3b) is also used as a plunger for removal of clot 38. In this embodiment stabilizing ring 27 is slidably mounted in area 18 positioned between inner catheter 14 and outer catheter 12. Movement of ring 27 is effected through the use of a user manipulated trigger mechanism. To remove clot 38 the user simply causes ring 27 to move towards opening 20. Such movement will dislodge clot 38. As an alternative, a plurality of stabilizing rings 27 may be positioned throughout area 18.

Stabilizing ring 27 allows component 26 to flow through area 18 unimpeded, however, stabilizing ring 27 is constructed in such a manner as to force clot 38 from opening 20 once stabilizing ring 27 has made contact with clot 38.

One such configuration is that of a spoked wheel (Figure 3b) wherein the spokes 33 of stabilizing ring or rings 27 define a plurality of openings 35. Openings 35 are large enough to allow component 26 to pass through while being small enough to force the removal of clot 38.

Alternatively, ring or rings 27 is a disk having a plurality of smaller openings 35 (Figure 3c).

Movement of stabilizing ring 27 is effected through the use of a trigger mechanism, such as a plunger or the equivalent thereof, positioned at the proximal end of applicator 10 which is conveniently accessed by the user's finger or thumb.

In yet another embodiment, ring or rings 27 are secured to inner catheter 14. Thus, as the user manipulates or moves inner catheter 14 ring 27 also moves.

In a still further embodiment, rings 27, or inner catheter 14 with rings 27 secured to it, are capable of being rotated as they are being depressed. Such rotation will enhance the dislodging of clot 38.

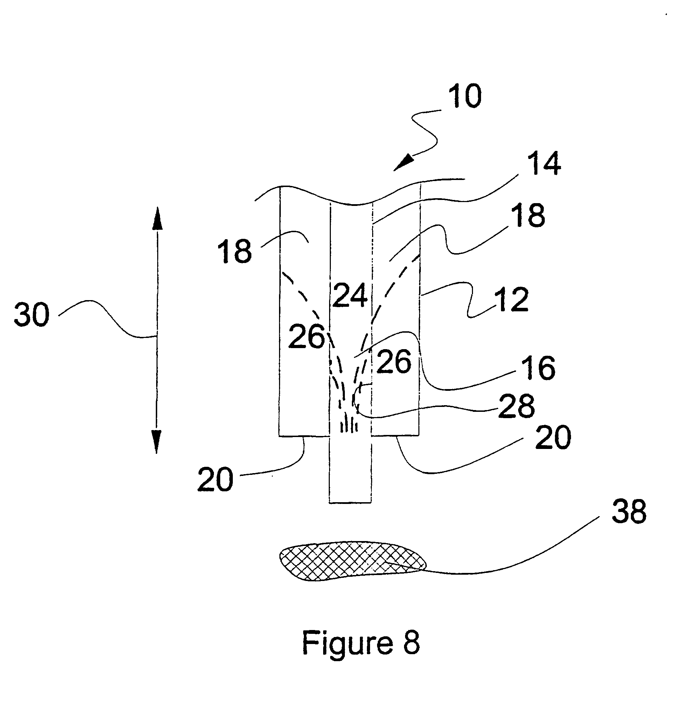

Other clot removal techniques such as those illustrated in Figures 7 and 8 may be utilized. In this sequence, a user first moves inner catheter 14 into the position illustrated in Figure 7. The movement will dislodge clot 38 from opening 28 of inner catheter 14. Then the user moves inner catheter 14 into the position illustrated in Figure 8. This movement allows inner catheter 14 to act as a plunger which will dislodge clot 38 from opening 20 thereby discharging clot 38 from applicator 10.

Alternatively, and as illustrated by the dashed lines in Figure 8. catheters 12 and 14 may be constructed out of a flexible material capable of being manipulated into the squeezed position illustrated by the dashed lines. This configuration allows the distal end of applicator 10 to be compressed to dislodge clot 38. In addition, small amounts of components 24 and 26 will be forcibly dispelled during this compression and will aid in the clearing of applicator 10. The manipulation of applicator 10 can be performed by the fingers of the user or a pinching mechanism which could be hand held or adhered to applicator 10.

Alternatively, catheters 12 and 14 may be constructed out of a flexible material capable of be manipulated at the proximal end of applicator 10 to effectively pinch off the supply of components 26 and 24. The compression of catheters 12 and 14 at the proximal end also causes small amounts of components 24 and 26 to be discharged. Such placement of a pinching mechanism or pinching capability at the proximal end of applicator 10 also provides for an ergonomic device.

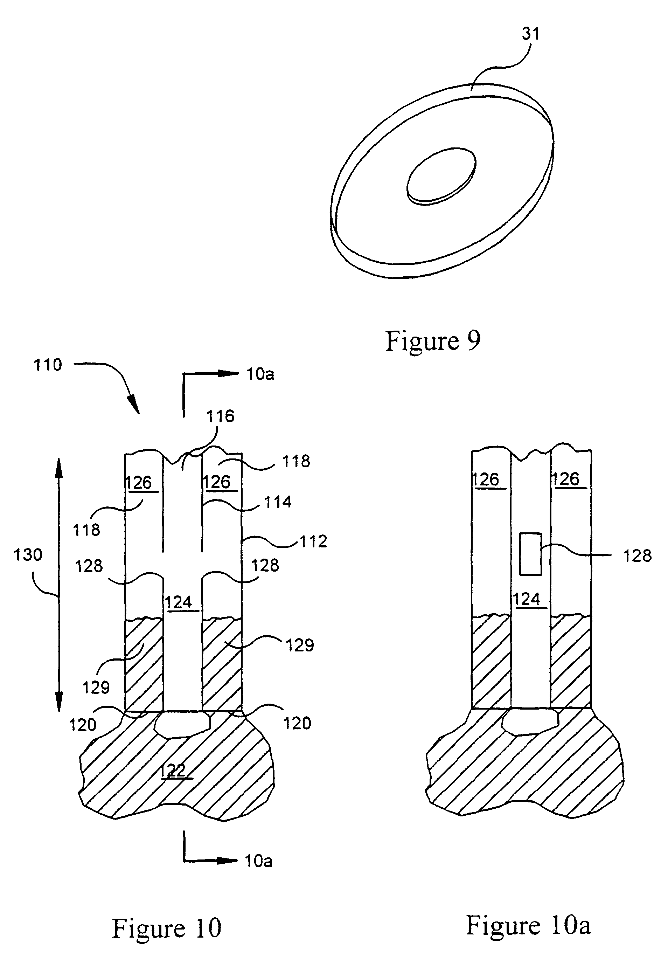

Openings 28 and 20 may be equipped with a closing mechanism, such as removable cover 31 (illustrated in Figure 9) which prevents unrestricted flow of components 24 and 26 from inner and outer catheters 12 and 14 between uses.

Referring now to Figures 10 and 10a an alternative embodiment of the present invention is illustrated. In this embodiment, components and/or parts performing analogous or similar functions are numbered in multiples of 100. Here an opening or plurality of openings 128 are positioned along the elongated portions or walls of catheter 114. These openings allow component 124 to mix with component 126 throughout an extended mixing volume 129 prior to discharge of mixed sealant 122 from opening 120. This embodiment allows for a particularly effective mixing of components 124 and 126 prior to application.

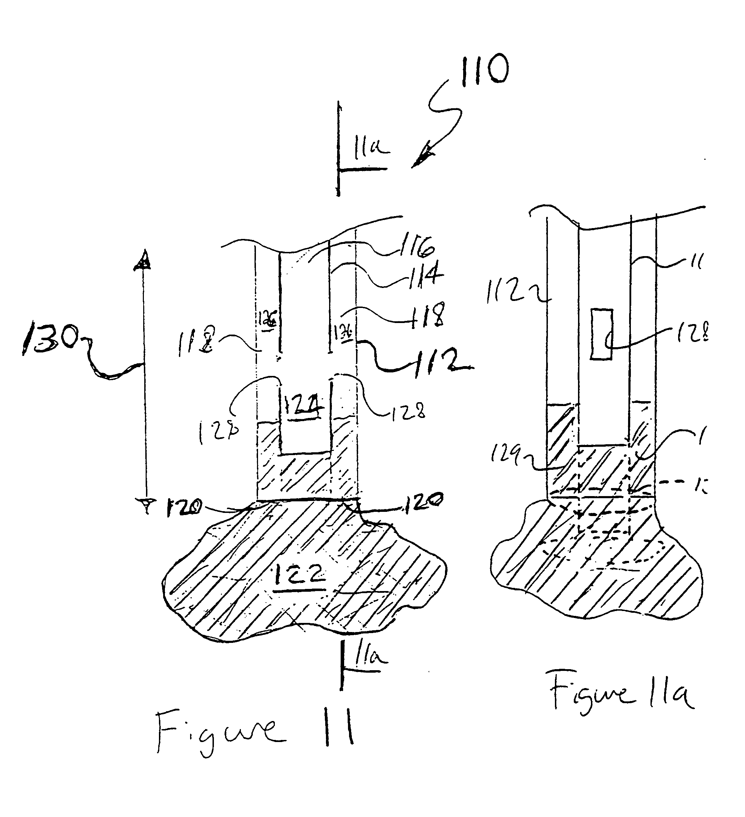

In this embodiment the end of catheter 114 is closed. Accordingly, mixed sealant 122 is applied to the required location through opening 120. Catheter 114 may also be positioned to apply sealant 122 as illustrated in Figures 11 and 11a.

In the case where a clot 138 has blocked opening 120, catheter 114 may also be used as a plunger to remove clot 138 (as illustrated by the dashed lines in Figure 11a).

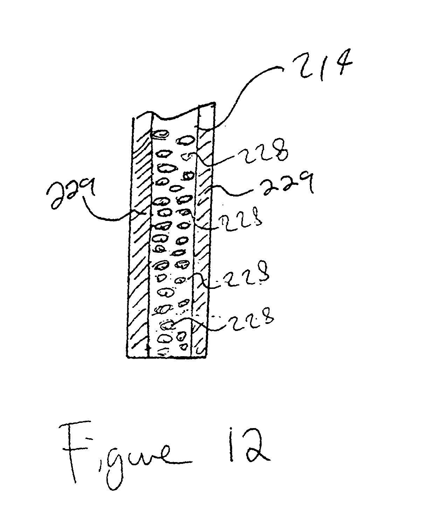

In yet another embodiment, and as illustrated in Figure 12, catheter 214 is configured to have a plurality of smaller openings 228 throughout a selected length of the distal end of the catheter 214, for example 1 or 2 cm., or the entire surface of catheter 214. This configuration allows for an even greater mixing volume 229. Preferably, sufficient pressure of sealant agent or component is maintained in inner catheter 214 to ensure avoid possible backflow and clogging of openings 228 owing to mixing of the sealant components in the openings.

In addition and in order to aid in the clot expulsion as depicted in Figures 6-8 small amounts of components 24 and 26 may also be expelled with the clot to ensure that all of the clot is removed from applicator 10.

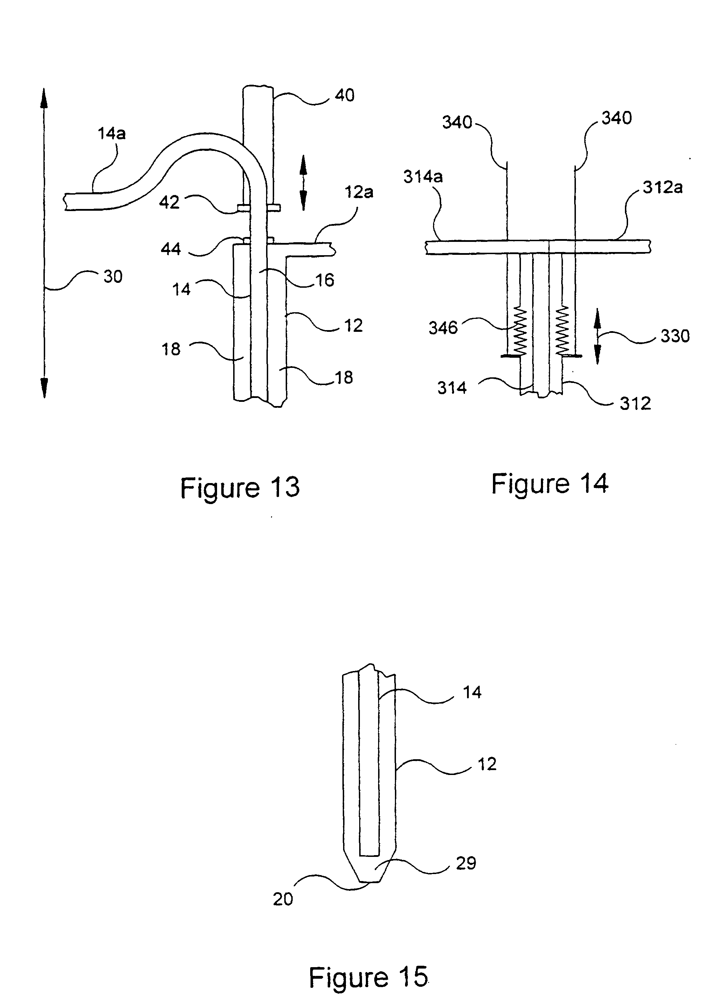

Referring now to Figure 13, one possible supply connection of inner and outer catheters 12 and 14 is illustrated. A flexible connector 14a is connected to the proximal end of inner catheter 14. Flexible connector 14a supplies area 16 with sealant component 24. The flexible nature of connector 14a allows inner catheter 14 to move in the directions of arrow 30.

Movement of inner catheter 14 is facilitated by the manipulation of an actuating mechanism 40 which is secured to inner catheter 14 via a clamping device 42. Such manipulation causes a corresponding movement of inner catheter 14, and may be effected by a manual control (not shown), for example a spring-loaded depressible button or trigger.

A flexible gasket 44 allows for movement of inner catheter 14 while providing a secure seal around catheters 14 and 12.

Outer catheter 12 is also connected to a connector 12a. Connector 12a supplies area 18 with sealant component 26. Connector 12a may also be flexible, however connector 12a may be constructed out of a more rigid material.

Referring now to Figure 14 yet another alternative embodiment of the supply connection of the present invention is illustrated. Here both inner and outer catheters 314 and 312 are connected to connectors 312a and 314a. In this embodiment catheter 312 is configured to have a flexible and expansible portion 346, which can, for example, be of fanfold or accordion-like bellows construction and is located intermediate to catheter 312 and connector 312a. Flexible portion 346 allows for constriction and expansion of outer catheter 312 in the directions depicted by arrow 330. Movement of catheter 312 is effectuated by an actuating mechanism 340. As mechanism 340 advances or retracts catheter 312 also moves in the same direction.

Referring now to Figure 15 an alternative embodiment of the distal, delivery end of applicator 10 is illustrated. Here catheter 12 is configured to have a tapered end, constricting the flow of sealant components to provide a mixing volume 29 with strong turbulence. The outer diameter of inner catheter 14 can be as large as opening 20, thus when inner catheter 14 is advanced, it exerts a precise plug-removal clearing action on the reduced diameter tip of outer catheter 12.

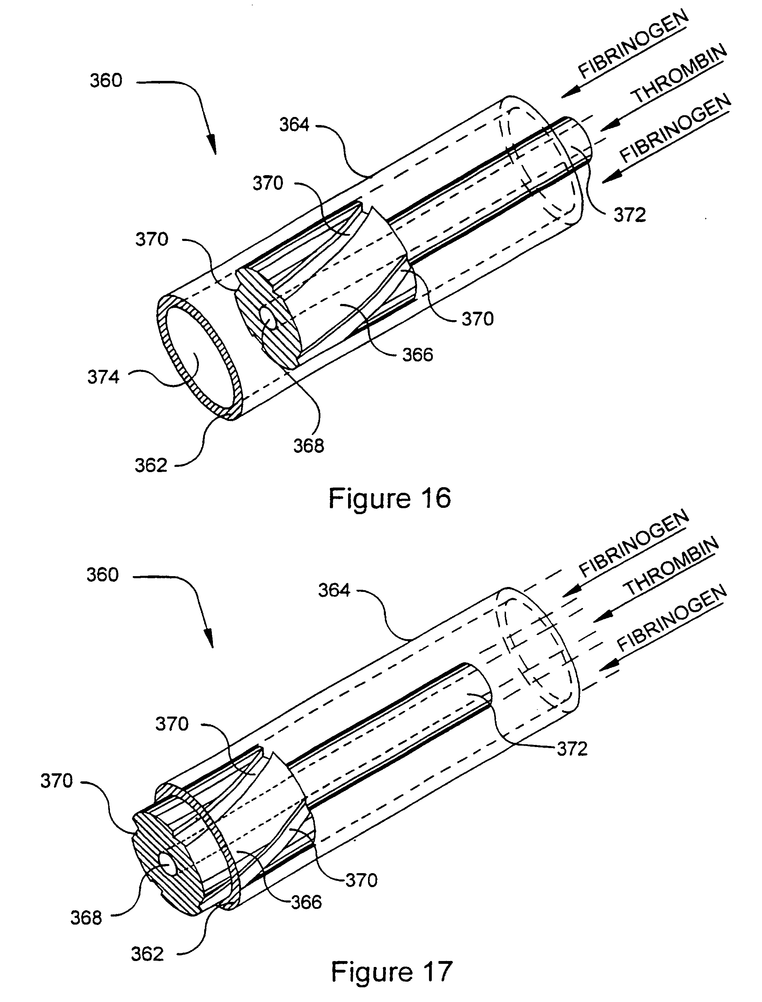

The embodiment of the invention shown in Figures 16-17, comprises a modified clearing-enabled, dual catheter sealant applicator 360 which can be fabricated from suitably soft or resilient materials, to have a flexible or malleable end or tip 362 to facilitate or enhance engagement of the applicator with, and entry of the applicator into, living tissues, for example, the ear, the nose or the throat. The material of applicator 360, at least at tip 362, should preferably be sterilizable and non-absorbent, as well as soft, or deformable, and resilient, and may for example, be a silicone rubber.

Catheter sealant applicator 360 comprises a hollow outer sheath 364, an inner plug 366 which has an axial bore 368 and external channels 370, and a supply lumen 372 press-fitted into plug 366. As shown, four symmetrically disposed channels 370 are provided, but other numbers of channels 370 such as three, six or eight may be employed. The outer diameter of plug 366 is a close sliding fit within the inner diameter of sheath 364. The cross-sectional areas of channels 370 and bore 368 are selected to provide desired relative flow rates of sealant agents, which relative flow rates may be varied, or selected, by using plugs 366 of different configurations. Such diverse plugs 366 may be supplied each with a lumen 372, or may be interchangeably fittable to one or more lumens 372. Optionally, channels 370 on plug 366 can taper outwardly, in the fluid delivery direction, which is to say distally, to increase the channel area in the direction of flow and facilitate clearing and the removal of clots.

A further option is for channels 370 to have a helical configuration, as shown, simulating rifling, to impart spin to the fluid travel to enhance mixing of the sealant agents. In such case a still further option is for channels 370 to be twisted in opposed directions around plug 366, to impart countercurrent flows and further enhance mixing.

Preferably, the sealant catalyst, for example, in the case of a fibrinogen sealant, thrombin or other fibrinogen activator, is supplied through lumen 372 to bore 368 in plug 366, and fibrinogen or other polymerizable sealant agent, is supplied in the space between the outside of the lumen and the inside diameter 374 of sheath 364.

For application of sealant, the forward or distal face of plug 366 is slightly retracted behind the distal end of sheath 364 to allow mixing of the two agents to take place in a mixing volume disposed generally within the sheath 364. As with other embodiments described herein, significant mixing of the sealant components takes place before delivery of the sealant agents to, or contact of the sealant agents with, the target work surface.

Once a cycle of sealant application is complete, tip 362 can be cleared of any clot or other material, by advancing lumen 372, driving plug 366 forwardly inside sheath 364 and expelling the clot. If necessary, the clot can be freed from the face of plug 366, or loosened, by dispensing a small amount of sealant.

While illustrative embodiments of the invention have been described above, it is, of course, understood that various modifications will be apparent to those of ordinary skill in the art. Many such modifications are contemplated as being within the scope of the invention, as defined in the appended claims.

## Claims

1. A multi-component sealant applicator (10) comprising a dual catheter for delivering sealant comprising a first catheter, a second catheter and further comprising a mixing volume (29) within the dual catheter for mixing multiple sealant components (24, 26) prior to discharge from a distal end of the catheter, wherein the first and second cathethers each communicate with one of a pair of fluid sealant agent sources, **characterised in that** the first catheter is configured to be movably mounted with respect to the second catheter.

2. An applicator (10) according to claim 1 wherein one catheter is mounted for longitudinal movement within the other.

3. An applicator (10) according to claim 1 further comprising a clearing system to clear undesired material from the mixing volume (29) or the vicinity of the mixing volume.

4. An applicator (10) according to any one of claims 1 to 3 wherein each catheter communicates with one of a pair of fluid sealant agent sources wherein one catheter is mounted for longitudinal movement within the other and the inner catheter (14) is usable as a plunger to remove clogs.

5. An applicator (10) according to claim 1 further comprising a stabilizer member to locate one catheter with respect to the other.

6. An applicator (10) according to claim 2 further comprising a stabitizer member to locate one catheter with respect to the other wherein the stabilizer member comprises a ring (27) disposed in the outer catheter (12) around the inner catheter (14) and provided with opening (35) for the passage of sealant component (26).

7. An applicator (10) according to any one of claims 1 to 3 further comprising a reciprocal drive mechanism proximally coupled with the dual catheter to move one catheter longitudinally back and forth with respect to the other.

8. An applicator (10) according to claim 7 wherein the drive mechanism comprises a ratchet and pawl.

9. An applicator (10) according to any one of claims 1 to 3 further comprising graduated markings (36) to indicate the relative position of one catheter with respect to the other.

10. An applicator (10) according to claim 6 further comprising a clot clearing mechanism operative to move the stabilizer ring (27) into engagement with a clot (38) to be cleared.

11. An applicator (10) according to claim 10 wherein the clot clearing mechanism comprises a manually actuatable trigger.

12. An applicator (10) according to claim 1 wherein the catheters have distal tips resiliently deformable to expel clots (38).

13. An applicator (10) according to claim 1 further comprising a cover (31) to close the distal end of the dual catheter.

14. An applicator (10) according to claim 2 wherein the inner catheter (14) comprises multiple lateral openings (128) in the vicinity of its distal end for flow of a sealant component to provide a mixing volume (129) in the outer tip.

15. An applicator (10) according to claim 14 wherein the end of the inner catheter (14) is closed.

16. An applicator (10) according to any one of claims 1 to 3 wherein the proximal ends of the catheters are coupled to sources of sealant components, one catheter having a flexible portion enabling it to be moved longitudinally relative to the other catheter.

17. An applicator (10) according to claim 16 wherein the one catheter is disposed in and surrounded by the other catheter for movement within the other catheter and is coupled to the other catheter through a flexible gasket (44) allowing for relative movement of the catheters and providing a fluid seal.

18. An applicator (10) according to any one of claims 1 to 3 wherein the proximal ends of the catheters are each coupled to a respective source of a sealant component and one catheter has a longitudinally expandable and contractible variable wall section permitting the length of the one catheter to be varied reciprocably with respect to the other whereby the distal end of the one catheter is longitudinally reciprocated with respect to the distal end of the other catheter.

19. An applicator (10) according to claim 18 further comprising a manually operable actuating mechanism (40) drivingly connected to the one catheter, at a point located distally of the variable wall section.

20. An applicator (10) according to any one of claims 1 to 3 wherein the one catheter is disposed in and surrounded by the other catheter for movement within the other catheter and has a distal tip which tapers distally.

21. An applicator (10) according to claim 1 wherein
a) the first catheter is an inner catheter (14) providing a flow path for delivering one sealant component (24);
b) the second catheter is an outer catheter (12) providing a flow path for delivering another sealant component, the inner catheter being disposed within the outer catheter; and
the inner catheter has a plug-like end being a close fit within the outer catheter and being provided with openings (128) for the passage of the other sealant component.

22. An applicator (10) according to claim 21 wherein the distal end of the outer catheter (12) comprises a malleable or resilient material to resist damage to biological tissue.

23. An applicator (10) according to claim 22 wherein the inner catheter (14) is reciprocably movable with respect to the outer catheter (12) to dislodge undesired solid material from the mixing volume (29).

24. An applicator (10) according to any one of claims 21 to 23 wherein the openings (128) in the plug-like end of the inner catheter (14) comprise channels formed in the outer surface of the plug.

25. An applicator (10) according to claim 24 wherein the channels extend laterally around the catheters to swirl liquid passing through the channels to enhance mixing.

## Patentansprüche

1. Applikator (10) für ein Mehrkomponenten-Dichtmittel, der einen Doppelkatheter zur Abgabe von Dichtmittel aufweist, der einen ersten Katheter und einen zweiten Katheter aufweist und der ferner einen Mischraum (29) in dem Doppelkatheter zum Vermischen einer Vielzahl von Dichtmittelkomponenten (24, 26) vor der Abgabe aus einem distalen Ende des Katheters aufweist, wobei der erste und der zweite Katheter jeweils mit einer von einem Paar von Quellen von fluidem Dichtmittel in Verbindung stehen,
**dadurch gekennzeichnet,**
**daß** der erste Katheter so ausgebildet ist, daß er in bezug auf den zweiten Katheter bewegbar angebracht ist.

2. Applikator (10) nach Anspruch 1,
wobei der eine Katheter für eine Längsbewegung in dem anderen angebracht ist.

3. Applikator (10) nach Anspruch 1,
der ferner ein Beseitigungssystem aufweist, um ein unerwünschtes Material aus dem Mischraum (29) oder der Nachbarschaft des Mischraums zu beseitigen.

4. Applikator (10) nach einem der Ansprüche 1 bis 3,
wobei jeder Katheter mit einer von einem Paar von Quellen von fluiden Dichtmitteln in Verbindung steht, wobei der eine Katheter für eine Längsbewegung in dem anderen angebracht und der innere Katheter (14) als Kolben verwendbar ist, um Klumpen zu entfernen.

5. Applikator (10) nach Anspruch 1,
der ferner ein Stabilisatorelement aufweist, um den einen Katheter in bezug auf den anderen zu lokalisieren.

6. Applikator (10) nach Anspruch 2,
der ferner ein Stabilisatorelement aufweist, um den einen Katheter in bezug auf den anderen zu lokalisieren, wobei das Stabilisatorelement einen Ring (27) aufweist, der in dem äußeren Katheter (12) um den inneren Katheter (14) herum angeordnet und mit Öffnungen (35) zum Durchtritt der Dichtmittelkomponente (26) versehen ist.

7. Applikator (10) nach einem der Ansprüche 1 bis 3,
der ferner eine hin- und hergehende Antriebseinrichtung aufweist, die proximal mit dem Doppelkatheter gekoppelt ist, um den einen Katheter in Längsrichtung in bezug auf den anderen hin- und herzubewegen.

8. Applikator (10) nach Anspruch 7,
wobei die Antriebseinrichtung eine Ratsche und eine Klinke aufweist.

9. Applikator (10) nach einem der Ansprüche 1 bis 8,
der ferner abgestufte Markierungen (36) aufweist, um die relative Position des einen Katheters in bezug auf den anderen anzuzeigen.

10. Applikator (10) nach Anspruch 6,
der ferner eine Klümpchenbeseitigungseinrichtung aufweist, die so wirksam ist,
daß sie den Stabilisatorring (27) in Eingriff mit einem zu beseitigenden Klümpchen (38) bewegt.

11. Applikator (10) nach Anspruch 10,
wobei die Klümpchenbeseitigungseinrichtung einen manuell betätigbaren Auslöser aufweist.

12. Applikator (10) nach Anspruch 1,
wobei die Katheter distale äußerste Enden haben, die elastisch verformbar sind, um Klümpchen (38) auszutreiben.

13. Applikator (10) nach Anspruch 1,
der ferner eine Abdeckung (31) aufweist, um das distale Ende des Doppelkatheters zu verschließen.

14. Applikator (10) nach Anspruch 2,
wobei der innere Katheter (14) eine Vielzahl von seitlichen Öffnungen (128) in der Nähe seines distalen Endes für den Durchfluß einer Dichtmittelkomponente aufweist, um einen Mischraum (129) in dem äußeren Ende zu bilden.

15. Applikator (10) nach Anspruch 14,
wobei das Ende des inneren Katheters (14) geschlossen ist.

16. Applikator (10) nach einem der Ansprüche 1 bis 3,
wobei die proximalen Enden der Katheter mit Quellen für Dichtmittelkomponenten gekoppelt sind, wobei der eine Katheter einen flexiblen Bereich hat, der es ermöglicht, daß er in Längsrichtung relativ zu dem anderen Katheter bewegt wird.

17. Applikator (10) nach Anspruch 16,
wobei der eine Katheter für eine Bewegung in dem anderen Katheter in dem anderen Katheter angeordnet und von diesem umgeben ist und mit dem anderen Katheter durch eine flexible Durchführungsdichtung (44) gekoppelt ist, die eine Relativbewegung der Katheter ermöglicht und eine Fluiddichtung bildet.

18. Applikator (10) nach einem der Ansprüche 1 bis 3,
wobei die proximalen Enden der Katheter jeweils mit einer entsprechenden Quelle einer Dichtmittelkomponente gekoppelt sind und der eine Katheter einen in Längsrichtung expandierbaren und kontrahierbaren variablen Wandbereich hat, der es ermöglicht, daß die Länge des einen Katheters in bezug auf den anderen hin- und hergehend verändert wird, so daß das distale Ende des einen Katheters in bezug auf das distale Ende des anderen Katheters in Längsrichtung hin- und herbewegt wird.

19. Applikator (10) nach Anspruch 18,
der ferner eine manuell betätigbare Betätigungseinrichtung (40) aufweist, die mit dem einen Katheter an einer von dem variablen Wandbereich distal befindlichen Stelle antriebsmäßig verbunden ist.

20. Applikator (10) nach einem der Ansprüche 1 bis 3,
wobei der eine Katheter für eine Bewegung in dem anderen Katheter in dem anderen Katheter angeordnet und von diesem umgeben ist und eine distales äußerstes Ende hat, das distal verjüngt ist.

21. Applikator (10) nach Anspruch 1, wobei
a) der erste Katheter ein innerer Katheter (14) ist, der eine Durchflußbahn zur Abgabe von einer Dichtmittelkomponente (24) bildet;
b) der zweite Katheter ein äußerer Katheter (12) ist, der eine Durchflußbahn zur Abgabe von einer anderen Dichtmittelkomponente bildet, wobei der innere Katheter in dem äußeren Katheter angeordnet ist; und
wobei der innere Katheter ein stopfenartiges Ende hat, das in den äußeren Katheter eng sitzend eingesetzt und mit Öffnungen (128) für den Durchtritt der anderen Dichtmittelkomponente versehen ist.

22. Applikator (10) nach Anspruch 21,
wobei das distale Ende des äußeren Katheters (12) ein plastisch verformbares und elastisches Material ist, um einer Verletzung von biologischem Gewebe entgegenzuwirken.

23. Applikator (10) nach Anspruch 22,
wobei der innere Katheter (14) in bezug auf den äußeren Katheter (12) hin- und herbewegbar ist, um ein unerwünschtes festes Material aus dem Mischraum (29) zu verdrängen.

24. Applikator (10) nach einem der Ansprüche 21 bis 23,
wobei die Öffnungen (128) in dem stopfenartigen Ende des inneren Katheters (14) Kanäle aufweisen, die in der äußeren Oberfläche des Stopfens gebildet sind.

25. Applikator (10) nach Anspruch 24,
wobei die Kanäle seitlich um die Katheter herum verlaufen, um durch die Kanäle hindurchtretende Flüssigkeit zu verwirbeln, um das Vermischen zu verstärken.

## Revendications

1. Applicateur de produit d'obturation à composants multiples (10), comprenant un cathéter double pour la distribution du produit d'obturation qui comporte un premier cathéter, un deuxième cathéter et qui définit en outre un volume de mélange (29) à l'intérieur du cathéter double pour mélanger les composants multiples (24, 26) du produit d'obturation avant la sortie de celui-ci à une extrémité distale du cathéter, dans lequel les premier et deuxième cathéters communiquent chacun avec une de deux sources de composant fluide du produit d'obturation, caractérisé en que le premier cathéter est configuré pour être monté de façon mobile par rapport au deuxième cathéter.

2. Applicateur (10) selon la revendication 1, dans lequel un premier cathéter est monté pour un mouvement longitudinal dans l'autre cathéter.

3. Applicateur (10) selon la revendication 1, comprenant en outre un système de nettoyage pour chasser une matière indésirable du volume de mélange (29) ou du voisinage du volume de mélange.

4. Applicateur (10) selon une quelconque des revendications 1 à 3, dans lequel chaque cathéter communique avec une de deux sources de composant fluide de produit d'obturation et dans lequel un cathéter est monté pour un mouvement longitudinal dans l'autre cathéter, et le cathéter intérieur (14) est utilisable comme plongeur pour chasser les bouchages.

5. Applicateur (10) selon la revendication 1, comprenant un élément de stabilisation pour positionner un cathéter par rapport à l'autre.

6. Applicateur (10) selon la revendication 2, comprenant en outre un élément de stabilisation pour positionner un cathéter par rapport à l'autre, dans lequel l'élément de stabilisation comprend un anneau (27) disposé dans le cathéter extérieur (12) autour du cathéter intérieur (14) et comportant des trous (35) pour le passage du composant de produit d'obturation (26).

7. Applicateur (10) selon une des revendications 1 à 3, comprenant en outre un mécanisme d'entraînement bidirectionnel couplé de façon proximale au cathéter double afin de déplacer un cathéter longitudinalement dans les deux sens par rapport à l'autre.

8. Applicateur (10) selon la revendication 7, dans lequel le mécanisme d'entraînement comprend un encliquetage.

9. Applicateur (10) selon une quelconque des revendications 1 à 3, comprenant en outre une échelle graduée (36) pour indiquer la position relative d'un cathéter par rapport à l'autre.

10. Applicateur (10) selon la revendication 6, comprenant en outre un mécanisme de nettoyage de caillot qui agit pour amener l'anneau de stabilisation (27) en contact avec un caillot (38) à éliminer.

11. Applicateur (10) selon la revendication 10, dans lequel le mécanisme de nettoyage de caillot comprend une gâchette actionnable à la main.

12. Applicateur (10) selon la revendication 1, dans lequel les cathéters ont des extrémités distales élastiquement déformables pour chasser les caillots (38).

13. Applicateur (10) selon la revendication 1, comprenant en outre un couvercle (31) pour fermer l'extrémité distale du cathéter double.

14. Applicateur (10) selon la revendication 2, dans lequel le cathéter intérieur (14) comprend des trous latéraux multiples (128) au voisinage de son extrémité distale, pour l'écoulement d'un composant de produit d'obturation, afin de créer un volume de mélange (129) dans l'extrémité extérieure.

15. Applicateur (10) selon la revendication 14, dans lequel l'extrémité du cathéter intérieur (14) est fermée.

16. Applicateur (10) selon une quelconque des revendications 1 à 3, dans lequel les extrémités proximales des cathéters sont raccordées à des sources de composants de produit d'obturation, un cathéter ayant une partie flexible qui permet de le déplacer longitudinalement par rapport à l'autre cathéter.

17. Applicateur (10) selon la revendication 16, dans lequel le premier cathéter est disposé dans l'autre cathéter et entouré par celui-ci pour un mouvement à l'intérieur de l'autre cathéter, et il est relié à l'autre cathéter par un joint d'étanchéité flexible (44) permettant un mouvement relatif des cathéters et créant une étanchéité aux fluides.

18. Applicateur (10) selon une quelconque des revendications 1 à 3, dans lequel les extrémités proximales des cathéters sont reliées chacune à une source respective d'un composant de produit d'obturation et un premier cathéter a une partie de paroi variable qui peut être augmentée et raccourcie longitudinalement pour permettre de faire varier la longueur de ce cathéter alternativement par rapport à l'autre cathéter, de sorte que l'extrémité distale du premier cathéter est déplacée longitudinalement en va-et-vient par rapport à l'extrémité distale de l'autre cathéter.

19. Applicateur (10) selon la revendication 18, comprenant en outre un mécanisme de manoeuvre actionnable à la main (40) connecté en entraînement au premier cathéter, à un endroit situé distalement par rapport à la partie de paroi variable.

20. Applicateur (10) selon une quelconque des revendications 1 à 3, dans lequel le premier cathéter est disposé dans et entouré par l'autre cathéter pour un mouvement à l'intérieur de l'autre cathéter, et il a une extrémité distale qui converge dans la direction distale.

21. Applicateur (10) selon la revendication 1, dans lequel;
(a) le premier cathéter est un cathéter intérieur (14) constituant un chemin d'écoulement pour la distribution d'un premier composant du produit d'obturation (24) ;
(b) le deuxième cathéter est un cathéter extérieur (12) constituant un chemin d'écoulement pour la distribution d'un autre composant du produit d'obturation, le cathéter intérieur étant disposé dans le cathéter extérieur ; et
le cathéter intérieur a une extrémité en forme de bouchon à ajustement étroit dans le cathéter extérieur et comportant des trous (128) pour le passage de l'autre composant du produit d'obturation.

22. Applicateur (10) selon la revendication 21, dans lequel l'extrémité distale du cathéter extérieur (12) comprend une matière malléable ou élastique qui évite d'endommager un tissu biologique.

23. Applicateur (10) selon la revendication 22, dans lequel le cathéter intérieur (14) est déplaçable en va-et-vient par rapport au cathéter extérieur (12) pour chasser une matière solide indésirable du volume de mélange (29).

24. Applicateur (10) selon une quelconque des revendications 21 à 23, dans lequel les trous (128) dans l'extrémité en forme de bouchon du cathéter intérieur (14) comprennent des canaux formés dans la surface extérieure du bouchon.

25. Applicateur (10) selon la revendication 24, dans lequel les canaux s'étendent latéralement autour des cathéters de manière à engendrer un mouvement de rotation du liquide qui passe dans les canaux, pour améliorer le mélange.
